(19) Europäisches Patentamt
European Patent Office
Office européen des brevets

(11) **EP 3 653 731 B1**

(12) **EUROPEAN PATENT SPECIFICATION**

(45) Date of publication and mention
of the grant of the patent:
**30.08.2023 Bulletin 2023/35**

(51) International Patent Classification (IPC):
**G16B 20/00** (2019.01)    **G16B 30/00** (2019.01)
**G16B 40/00** (2019.01)    **C12Q 1/6869** (2018.01)
**G16B 40/10** (2019.01)    **G16B 20/20** (2019.01)

(21) Application number: **19216343.4**

(22) Date of filing: **27.12.2016**

(52) Cooperative Patent Classification (CPC):
(C-Sets available)
**G16B 20/00; C12Q 1/6869; G16B 20/20;
G16B 30/00; G16B 40/00; G16B 40/10**    (Cont.)

(54) **METHOD FOR PERFORMING GENOTYPING ANALYSIS**

VERFAHREN ZUR DURCHFÜHRUNG VON GENOTYPISIERUNGSANALYSEN

PROCÉDÉ PERMETTANT D'EFFECTUER UNE ANALYSE DE GÉNOTYPAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**20.05.2020 Bulletin 2020/21**

(62) Document number(s) of the earlier application(s) in
accordance with Art. 76 EPC:
**16306825.7 / 3 343 415**

(73) Proprietor: **Limagrain Europe
63360 Saint-Beauzire (FR)**

(72) Inventors:
• **DUMAS, Anne-Valérie
63200 Mozac (FR)**
• **CHAUVET, Stéphanie
63170 Aubiere (FR)**
• **GUILLOUD, Sandra
63540 Romagnat (FR)**
• **MARTINANT, Jean-Pierre
63910 Vertaizon (FR)**
• **COLLANGE, Guillaume
63170 Aubiere (FR)**

(74) Representative: **Flesselles, Bruno F.G.
BF IP
36 rue Jean de la Fontaine
75016 Paris (FR)**

• **FU C-P ET AL: "Inferring ancestry in admixed
populations using microarray probe intensities",
PROCEEDINGS OF THE ACM CONFERENCE ON
BIOINFORMATICS, COMPUTATIONAL BIOLOGY
AND BIOMEDICINE, BCB '12, 1 January 2012
(2012-01-01), pages 105-112, XP055386009, New
York, New York, USA DOI:
10.1145/2382936.2382950 ISBN:
978-1-4503-1670-5**
• **DIDION J P ET AL: "Discovery of novel variants
in genotyping arrays improves genotype
retention and reduces ascertainment bias", BMC
GENOMICS, BIOMED CENTRAL LTD, LONDON,
UK, vol. 13, no. 1, 19 January 2012 (2012-01-19),
page 34, XP021117835, ISSN: 1471-2164, DOI:
10.1186/1471-2164-13-34**
• **SARGOLZAEI M ET AL: "A new approach for
efficient genotype imputation using information
from relatives", BMC GENOMICS, BIOMED
CENTRAL LTD, LONDON, UK, vol. 15, no. 1, 17
June 2014 (2014-06-17), page 478, XP021189033,
ISSN: 1471-2164, DOI: 10.1186/1471-2164-15-478**
• **PIRANI A ET AL: "Best practices for genotyping
analysis of plant and animal genomes with
Affymetrix' Axiom Arrays", , 1 January 2013
(2013-01-01), XP055385422, Retrieved from the
Internet:
URL:http://tools.thermofisher.com/content/
sfs/posters/Affymetrix-Best-practices-for-
plant-and-animal-genotyping-Ali-Pirani-201
3.pdf [retrieved on 2017-06-27]**

(56) References cited:
**US-A1- 2014 114 582    US-A1- 2014 274 749**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DOUGLAS J A ET AL: "Probability of Detection of Genotyping Errors and Mutations as Inheritance Inconsistencies in Nuclear-Family Data", AM. J. HUM. GENET, vol. 70, 1 January 2002 (2002-01-01), pages 487-495, XP055385798,**

(52) Cooperative Patent Classification (CPC): (Cont.)

C-Sets
**C12Q 1/6869, C12Q 2537/165**

**Description**

The invention relates to a computer implemented method to improve the reliability of the information generated during high-throughput genotyping, in order to be able to assign reliable allele information to genotyped individuals. Such methods are critical in breeding activities. Using Genotyping data in breeding schemes means to be able to manipulate high quality big data in a short delay. These methods make this task possible. High throughput genotyping processes require high throughput analytical methods.

As indicated in Lin et al (Bioinformatics (2008) 24 (23): 2665-2671), *"Single nucleotide polymorphisms (SNPs) are DNA sequence variations that occur when a single nucleotide (A, T, C or G) in the genome sequence is altered. [...] The vast majority of SNPs are biallelic. Consider a SNP marker with alleles A and B. There are three possible genotypes for a disomic individual, AA, AB and BB. Many low- and high-throughput technologies have been developed to genotype the SNPs efficiently, including the GeneChip Human Mapping Array from Affymetrix, the Illumina platform, the Sequenom platform, the Taqman platform and the Invader assay. Each platform uses a different technology, and they give somewhat different forms of data. In general, they all give certain quantitative measures of allelic abundance for the two alleles, yA and yB. The abundance measures can either be scalars or vectors. Individuals with genotype AA are expected to have high yA value and low yB value. The opposite is expected for individuals with genotype BB. Those with genotype AB are expected to have similar yA and yB values."*

The results of the measures on the array can be plotted on two dimension graphs for each individual and each marker.

*"Each dot on the plot represents one individual. In SNP genotyping, we seek to identify genotype clusters based on these measurements and 'call' each person's genotype by assigning them to a cluster. Normally, we expect to find three clusters, but if one allele is rare in the population, a particular dataset might only have two clusters (genotypes)"* Lin et al (Bioinformatics (2008) 24 (23): 2665-2671).

The development of high-throughput genotyping methods, such as the one developed by Affymetrix and Illumina makes it possible to simultaneously obtain genotyping information for a large number of individuals and multiple markers.

To obtain this information, manipulation steps are directly performed in the laboratory: sample preparation, DNA extraction, and run on arrays or chips where markers are present.

Raw data files (such as .CEL files in the Affymetrix process) are obtained after the run on the chip. These raw data contain the raw result (such as fluorescence intensity and data) for each individual and each marker.

The raw data files are then analyzed, using computer devices and software that assign (or try to assign) specific allele information to each individual for each marker.

As indicated above, for each marker, these softwares will generally assign an allele to an individual and thus create clusters of individuals (regrouped by allelic information). The quality of the assignment is generally checked and confirmed through the use of various indicators.

The final output would be a data matrix containing alleles for all individuals and all markers.

**Description of the prior art process**

This translation process is to analyze the raw data (fluorescence data) of an individual and interpret it so as to obtain allelic marker information associated with each and every individual.

The principle is generally as follows: for a given marker, an allele (such as AA, BB for homozygous individuals, or AB for heterozygous individuals) is assigned to an individual based on the raw signal values. The signals associated with each individual can be plotted on a two-dimensional graph wherein each of the individuals having alleles AA, (resp. AB or BB) are localized in the same zone (generally an elliptic zone), that is named cluster AA (resp. AB or BB).

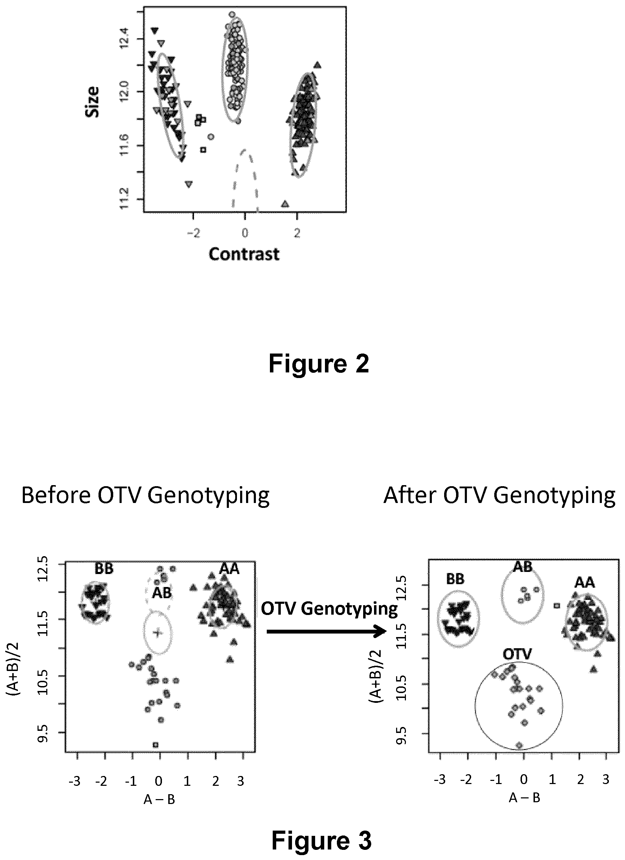

Such representation and clustering can be performed using any software known in the art, such as the Axiom Analysis Suite Version 1.1. (Figure 2).

Tools (based on mathematical modeling tools) for assigning an allele to each "individual / marker" pair are known in the art and provided by the manufacturers of the genotyping solutions (such as the manufacturers of the microarrays). In particular, Affymetrix provides algorithms based on models that are described in the literature (BRLMM: an Improved Genotype Calling Method for the GeneChip® Human Mapping 500K Array Set Revision Date: 2006-04-14 Revision Version: 1.0; BRLMM-P: a Genotype Calling Method for the SNP 5.0 Array Revision Date: 2007-02-13 Revision Version: 1.0 ; Birdseed). These tools may be parameterized by the user.

In other technologies, such as the technology developed by Illumina, the translation of raw data allelic data is facilitated by the use of "masks". The raw data is imported within the software, which will perform automatic reading (automatic assignment of an allele for each pair individual / marker). From these readings, the end-user can save the equations of the ellipses corresponding to the position of alleles clusters for each event, in so-called "cluster file". This information ("mask") can then be used when new individuals are to be genotyped.

It is to be noted, however, that the ellipses equations are fixed and that it is difficult to make them evolve and to customize them during analysis of the samples. Consequently, since the position of the clusters (equation of the ellipses) depends on the type and origin of the material that is genotyped (grain, leaf, grain mixture thereof...), this technique may prove not to be appropriate to sample variability in all cases.

A mask should thus be created for each type of material to be genotyped which is time and resource consuming. Furthermore, even though these masks help with assignation of a given allele to a given individual for a given marker, verification and manual correction for some markers are still needed (markers with low CallRate or aberrant level of heterozygosity).

*Translating raw data into allelic data for a sub-set of markers*

This step is generally first performed on a subset of markers in order to optimize the assignment for all individuals.

*Filter individuals for reliable process*

Sample quality may also affect genotyping. In particular, if, in a batch of individuals, one or more samples are of poor quality, their presence can impact the quality of genotyping for all individuals of the batch. Indeed, presence of these individuals would move the localization of the clusters and hence lead to wrong assignment of alleles for some individuals.

The quality can be checked using various indicators that are known in the art.

The dQC (dish quality control) is an indicator used in Affymetrix solution to make it possible to detect contamination problems, taking both interchannel and intrachannel signal separation.

A commonly used indicator is the CallRate. At the individual level, it measures the percentage of markers for which allelic data could be obtained (i.e. an allele has been assigned to the individual for the marker). Consequently, the overall call rate of a sample is equal to the number of markers where receiving an AA, AB, or BB allelic genotype is assigned divided by the total number of markers on the chip.

A low CallRate for an individual may be due to various reasons, such as contamination of the sample or poor DNA concentration or poor quality DNA. It is therefore recommended not to keep the information associated with the individuals with a low CallRate.

More generally, it is recommended to remove the raw data for the low CallRate individuals before performing the genotyping analysis of the other individuals. Indeed, as indicated above, the presence of low quality individuals may add to the variability at the time of modeling (determination of the clusters) and thus impact the quality of allele assignment as a whole.

*Translating raw data into allelic data for all markers*

After the previous step, translating raw data into allelic data is done for all markers. This step is performed as indicated above for the sub-set of markers, the differences lying in the fact that it is performed for all markers on individuals that have passed the quality control thresholds (such as sufficiently high dQC and CallRate).

*Qualitative classification markers*

Once allelic data has been obtained for all selected individuals, one can use scripts to classify markers so as to keep only the allelic data corresponding to the most reliable markers.

As an illustration, Affymetrix proposes the software SNPolisher_1.3.6.6, GTC 4.1.4, described in Best Practices Workflow and SNPolisher for Custom Axiom Array Analysis, March 2013). Later version of the SNPolisher software can be found at http://www.affymetrix.com/estore/partners_programs/programs/developer/tools/dev nettools.affx

Using these softwares, the markers can be classified into six groups:

- "PolyHighResolution" (well defined clusters),
- "MonoHighResolution" (only one cluster),
- "Off-Target Variant" (presence of a cluster "absence of signal"),
- "No Minor Hom" (one homozygous cluster is missing),
- "Call Rate Below Threshold" (with other indicators above threshold) and
- "Other".

The "PolyHighResolution" markers correspond to the most reliable markers (for which the allele assignment is the most reliable).

The markers and their quality can be ranked using various indicators.

In particular, Affymetrix uses the indicators SNP Call Rate (percentage of individuals for which an allele was assigned for the given marker), FLD (Fisher's Linear Discriminant), HetSO (Het Strength Offset), HomRO (Homozygous Ratio Offset), as described in Analysis Guide, Axiom Genotyping Solution Guide Data Analysis, available at http://media.affymetrix.com/support/downloads/manuals/axiom_genotyping solutio n_analysis_guide.pdf)

It is possible to find other indicators in the literature, such as the Silhouette index (Rousseeuw (1987) "Silhouettes: a Graphical Aid to the Interpretation and Validation of Cluster Analysis." Computational and Applied Mathematics 20: 53-65), or the Davies Bouldin Index (Davies and Bouldin (1979) "A Separation Cluster Measure" IEEE Transactions on Pattern Analysis and machine Intelligence PAMI-1 (2): 224-227).

Figure 3 illustrates the analysis of a marker which is qualified as OTV marker.

The purpose of all these tests and controls is to identify markers for which the clusters that correspond to the different alleles are clear (i.e. AA, AB and BB alleles groups are clearly separated from each other) and thus reliable.

*New translation when justified*

As indicated above (and illustrated in Figure 3), some markers are classified as PAV markers (Presence Absence Variant), also named OTV (Off Target Variant). This may be due to an absence of the allele or presence of an allele that is significantly different from the sequences of the probes on the array.

For these markers, one would find four clusters corresponding to alleles AA, BB, AB and Absence of Signal.

Affymetrix provides a tool adapted to these specific cases, namely a R function called OTV_Caller, which is known and available in the R SNPPolisher package provided by Affymetrix.

The publication "Statistical methods for off-target variant genotyping is Affymetrix'Axiom® Arrays," Teresa A. Webster, Alia Pirani, Mei-mei Shen, Laurent Bellon and Hong Gao describes this process.

Actually, analysis of these specific markers requires an extra step, which is to identify an allele class (cluster) that is graphically below the other alleles classes, and is sufficiently homogeneous and dense to be considered a true cluster and real signal rather than background noise.

Once this new cluster has been identified and qualified for a specific marker, the allele "absence of signal" can be assigned to the individuals in this cluster and the marker will thus be classified as a PAV or OTV marker.

*Quality control*

Quality control is performed, for example through the use of commercially scripts such as the ones provided by Affymetrix via the Ps_Metrics function of the SNPolisher package.

In brief, this will generate reports containing the Call Rate and heterozygosity rate information, class of markers and the values associated with each indicator that were used to classify the markers.

Depending on predetermined thresholds, alerts can be provided to the user on reliability of the data provided for a given marker.

**Issues associated with the current methods**

A major challenge of the high throughput genotyping techniques is to maximize the quality of allelic information obtained in the end, in order to optimize the quantity, reduce costs and time associated with these processes.

Currently the problems comprise:

- The analysis is generally specific to the technology used (Affymetrix, Illumina) and to the robots that generate the raw data. In consequence, one must adapt to new tools as soon as it incorporates a new technology.
- There is a need to have a "stable set of reliable markers of good quality" for which one can be sure of reliability of the information obtained. Currently a reliable marker set (that would provide reliable information for all individuals) changes every time a genotyping chip is used with a new set of individuals or a different technology.

When one wishes to compare two individuals whose genotyping was obtained within different runs, the set of markers available and of good quality for both individuals will be limited and will thus limit the genetic interpretation of results. This problem will be as important as the number of studied individuals is important.

- There are usually some errors in the automatic classification of markers as described above, and manual checks are necessary.
- The quality control tools as described above that are present in the solutions developed by the technology providers are limited and do not guarantee that the genotyped material corresponds to the expected material (that there was no contamination or exchange of plates) or to diagnose any problems during the genotyping process.

It is thus necessary to improve the process of allocating alleles to individuals, to improve the quality control, in particular to improve the reliability of the markers that will be used, to obtain a method that would be less experiment-dependent.

Obtaining high quality marker data is critical for all marker-assisted selection applications. Low quality data will decrease the efficiency of plant breeding.

The above-process will thus be modified at multiple steps, that can be used independently, or in any combination:

(a) Use of a reference panel to obtain a first representation of allele-clusters, so that it is possible to adapt the settings of the analysis software and increase reliability of cluster assignment

(b) Optimization of the number of reliable markers by the use of new quality control indicators

(c) Correction of interpretation / Determination of PAV markers

(d) Control of consistency of results / quality controls, in particular by using new indicators that are indicative if inconsistency

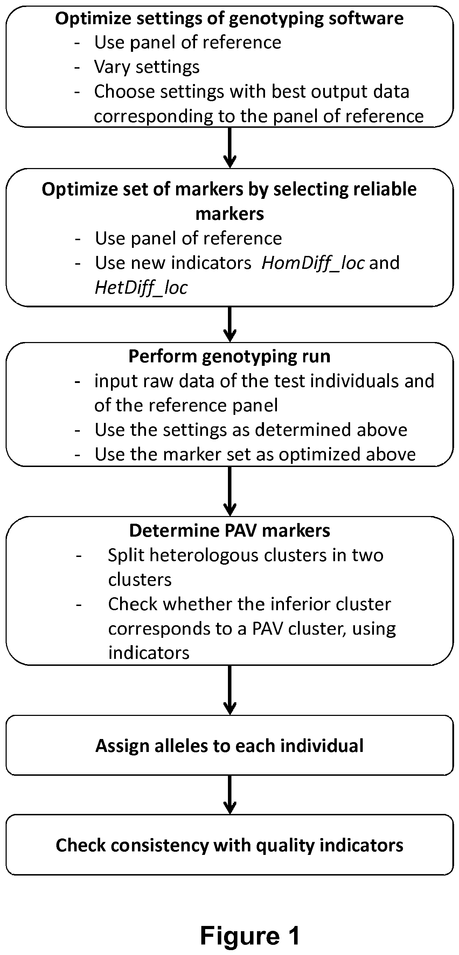

Such process is illustrated on Figure 1.

It is however preferred when all these steps are combined together as this will largely improve the quality of the data obtained, as well as the quantity of information obtained (increase in the number of markers for which reliable information is obtained).

This process can be implemented within a software comprising a combination of optimized library which can accept, as input, any raw data, whatever the genotyping method used to obtain this raw data, and will provide, as output, visualization of the clusters, and any other information such as the genotyping information for the markers of the genotyping run and the individual tested in the run, allele calling, quality status and flags, tools for decision making.

**Definitions**

An "allele" is one of a number of alternative forms of the DNA sequence at a given genetic locus.

An "array" or a "chip" is a DNA microarray which is used to detect polymorphisms within a population. It operates with

- an array containing immobilized nucleic acid sequence specific to a genomic position of interest
- solution probes or nucleic acid labeled with fluorescent dyes
- And fragmented nucleic acid sequences of individuals that need to be genotyped.
- A detection system for fluorescent signal witch create the "raw data" (hybridization signals obtained for each marker and each individual).

A "cluster" is the visual representation of the fluorescence signal on a two-dimentional graph for a group of individuals having the same genotype for a given marker. It is usually represented as an ellipse when the individual measures out of the chip are plotted on a two-dimensional graph.

An "individual" is a plant that is included in a genotyping program, and from which DNA is isolated and genotyped according to methods herein disclosed,

A "locus" is the specific location of a DNA sequence on a chromosome.

A "molecular marker" or "marker" is a gene or DNA sequence with preferably a known location on a chromosome for which different alleles can be revealed through the use of molecular protocols. It can be a short DNA sequence, such as a sequence surrounding a single base-pair change (single nucleotide polymorphism, SNP), or a long one, like microsatellites.

In summary, a marker makes it possible to discriminate between different alleles at a specific locus. Use of markers to do so in an individual consists in genotyping said individual (see below). An individual for which the alleles at different loci have been determined is said to be a "genotyped individual" for the markers associated with these loci.

A "run" is the part of genotyping experiment that consists of preparing the samples, applying them to the hybridization device, and acquiring the hybridization signal.

"Raw data" is what is obtained after performance of a run. It consists of the hybridization signals obtained for each marker and each individual.

A "program" is a "genotyping experiment", including the run and the allocation of alleles to the individuals.

"genotyping" means determining the combination of alleles for one marker or a set of markers. In the context of the present invention, genotyping may be performed on the whole genome of an individual, on a part of the genome, such as on one or more chromosomes, on a part of one or more chromosomes, or on one or more specific regions of the genome as for example a gene. A lots of genotyping techniques exists, such as the GeneChip Human Mapping Array from Affymetrix, the Illumina platform, the Sequenom platform, the Taqman platform and the Invader assay. A synonym of "genotyping" in the context of the invention can be "allele calling".

In order to genotype an individual (or part of the genome of an individual), one uses a set of markers. By extension, sequences that may be polymorphic between different individual can be named "markers" (it is clear that polymorphism for a given marker can be observed between a specific individual and a second individual, whereas it would not be observed with a third individual). These markers can thus indicate the nature of an allele at the specific locus that they target.

For markers that differentiate between two alleles, and assuming that the frequency of both alleles is balanced, it is statistically assumed that about 50 % of the markers will reveal the same allele, between two unrelated individuals, just by chance.

A "genotyping experiment" comprises the steps of preparing samples of the individuals, obtaining raw data, analyzing the raw data, and assigning alleles to the individuals.

A "genotyping software" is a software that is used to allocate alleles to individuals after a genotyping run, by analyzing the raw data (hybridization signals) obtained for the individuals for each marker, that are used as input within this genotyping software. Such software are known in the art and are usually provided by the companies having developed the genotyping technology.

A "statistical software" is a software that makes it possible to do statistical analysis after input of data (such as the R software, available at https://www.r-project.org/)

A "parent" for an individual is, in the context of the invention the, or one of the, donor(s) of marker alleles of this individual. Several generations of crossing, re-crossing, selfing (self-pollination) or other steps (like the ones involved in doubled haploid production process) could have occurred between the parental stage and the individual stage but in all cases the alleles of one individual belong to the set of alleles of its parents.

*Use of a reference panel to obtain a first representation of allele-clusters*

In this step, the "raw data from a reference panel" that contains individuals, the genotype of which is known, is used.

In the context of the invention, the raw data of the reference panel is analyzed together with the raw data of the alleles of the genotyping experiment.

This reference panel consists of a set of individuals selected to maximize the presence of several individuals (5 minimum) in each one of the three clusters for each of the selected marker.

The selection of these individuals is described below, but it is preferred that these individuals are chosen, for example in different genetic groups for maize. In addition, the individuals constituting the panel are selected so that the clusters associated with each of the alleles exist for all of the markers and that these are clearly identified for each marker for these individuals, The method does not check if some individuals correspond to the absence of signal The method only consider the presence of at least 5 individuals in each one of the three main clusters.

This panel may for example consist of 384 individuals (as experiments are generally made using 384-well plates), so that it can be considered as an independent genotyping experience. One can use fewer or more individuals, depending on the number of markers studied, or of the species studied.

Individuals that are used in the reference panel have previously been "robustly" genotyped, *i.e.* that have been genotyped more than once, with always the same result obtained, or that are progeny of parents for which the genotype is known. Consequently, the alleles for the individuals of the reference panel are known.

It is to be noted that the step according the present method does not require performing actual experiments on the genetic material of the individuals of the reference panel. The present method can use the raw data that has been previously generated for these individuals (reference panel).

In the present method, the raw data file (such as the file with the extension .CEL, when generated through an Affymetrix genotyping process) associated with this reference panel is introduced within the softwares of the invention that assign an allele to each "individual / marker" pair.

One of the purpose of using the reference panel is to optimize the settings/parameters of the software to later reliably analyze the raw data obtained from individuals that one wants to genotype. Since the alleles of the individual of the reference panel are known, one will be able to finely design the locations where the clusters are expected for all the considered markers.

In order to do so, one will modify various setting/parameters available in the software and compare the genotyping data (allele allocation for each marker) obtained using these different configurations and options.

In particular, one can modify the parameters *"Option Inbred Penalty"* and *"genotype option"* that are used and disclosed in the Affymetrix MANUAL: apt-probeset-genotype (1.19).

This manual is, in particular, available at http://www.affymetrix.com/estore/su pport/ developer/powertool s/ch angelog/apt-probeset-genotype.html.affx

The *"Option Inbred Penalty"* takes into account the information relating to the heterozygosity rate expected for each individual.

The *"genotype option"* provides information on the expected alleles for some pair (individual / marker) to the algorithm / software. The algorithm takes into account this information, although the algorithm is able to provide output data different from the information already known for the reference panel, if this information seems not consistent with the actual positions of the observed clusters.

Once all the configurations have been tested, it is possible to determine which configuration provides the best output data, as compared to the data known for the individuals of the reference panels.

The best settings/parameters for the software can thus be saved. The allelic determination for the reference panel using these setting/parameters are recorded on a filed named *"genotype file",* this file containing the real allele data for each individual and each marker from the reference panel.

The two files associated with the reference panel individuals (raw data file and corrected allelic data file *(genotype file)* obtained as described above), are then incorporated in routine with each new genotyping experiment on the same species, containing common markers or individuals).

It is to be noted that one can also obtain a *"Posterior file"* (when using the Affymetrix APT-PROBESET-GENOTYPE algorithm) which contains the equations of the ellipses (clusters) corresponding to each allele class for each marker. This file corresponds to the *"mask"* described above for the Illumina system. This *"Posterior file"* can also be used in future genotyping runs.

In summary:

- One elaborates a reference panel containing individuals whose genotype is known.
- The raw data from this reference panel is inserted within the analysis software, and various settings/parameters are tested in order to identify those given the best interpretation (allele allocation) for the raw data ;
- The genotyping data thus obtained is extracted in a *"genotype file";*
- The optimized equation of the ellipses / localization of the allele for the markers extracted in a *"posterior file".*
- These two files thus generated as well as the raw data file, are then used in routine, *i.e.* the raw data file from the reference panel is inserted with the raw data file of the individuals to genotype and the two other files *(genotype file and posterior file)* are used as input for the software
- The best settings/parameters are used for the software.

This step thus corresponds to a method for obtaining parameter data that will be used in a computer software for analyzing raw data and assigning allelic information to said raw data, wherein said computer software preferably comprises a visualization interface of said genotyping data,
comprising the steps of

(a) introducing raw data from a reference panel as an input in said computer software, and wherein the allele information is known for the individuals of said reference panel
(b) varying the settings/parameters of said computer software so as to determine the setting/parameters that allow the best assignment of alleles for each individuals of said reference panel
(c) extracting said settings/parameter data that comprise computer files said the equations of said ellipses as determined in (b) and of the genotyping data associated, for each marker, with each individual of said reference panel.

The raw data of step (a) is the data obtained from the chip / array, containing the markers, and on which the physical samples have been applied. This raw data is in the form of a computer file that will contain, for instance and depending of the technology used, fluorescence intensity for each individual and each marker that depends on the level of interaction of the allele of the individuals with the probe on the chip/array. These files are generated by the devices sold by the chip/array/device providers and are in the adequate form to be inserted within the analysis software.

As indicated above, the user has some latitude to make the analysis parameters vary, for each marker. Step (b) uses this latitude in order to obtain the parameters that will optimize the quality (in particular the global CallRate).

Step (c) corresponds to the recording of the optimized setting/parameters that have been confirmed to provide good output data for the reference panel (which, as indicated, is composed of individuals for which allelic information is known), and that can thus be relied on to provide good information for other individuals of the same species that will be genotyped within the same program or by the same technology than the reference panel.

This method is a computer implemented method. The visualization makes it possible to see, for each marker, the plotting of each individual genotyping result [i.e. plotting, for each marker, the signal as determined for each individual during the run on the chip, on X/Y axis]. This visualization interface can further comprise the drawing of ellipses that represent the allele clusters associated with said marker. This plotting is known in the art. This software can be associated to other genotyping analysis tool, and the plotting of each individual genotyping result can be sent directly to these tools for further processing, if the quality controls are all positive for the experiment.

It is thus possible to perform, in this context, a method for genotyping a population of test individuals with a computer software for generating genotyping data, usually from raw data or after high throughput generation of data, comprising the steps of

(a) inputting within said computer software

  ∘ the raw data from said test individuals
  ∘ the raw data from a reference panel
  ∘ the setting/parameter data as obtained by the method disclosed above from said reference panel

(b) Running said computer software with said setting/parameter data so as to obtain genotyping data for said test individuals.

The genotyping data thus obtained is the allelic information for each marker and each individual.

The reference panel has also a second purpose. As the raw data for this reference panel is to be introduced in each run, the assignation of the alleles for this reference panel can be checked and used as a control: indeed, if the only clear assignation of alleles is obtained for the data from the reference panel, this would strongly suggest that there was some kind of dysfunction in the production of the data for the test individuals, such as chip degradation, reactive deficiency or degradation of the materiel. Such method will prevent generation of poor quality data.

*Choice of the reference panel*

In a preferred embodiment, said individuals in said reference panel have been chosen according to a method comprising the steps of:

(a) Determining the minimal number of individuals (n) that need to be present in each cluster. Usually this minimum number must be of 5.
(b) Selecting n random individuals from a starting panel for which the allelic information is known for each individual
(c) Adding one new individual from said starting panel, wherein said added individual is chosen so as to most increase the number of clusters that respond to the desired condition (at least n individuals per cluster)
(d) Repeating step (c) until the desired condition (at least n individuals per cluster) is met.

**Determination of marker quality before the run and selection of reliable markers**

The above-mentioned process for determining parameters and settings also allows the optimization of the number of markers that are correctly and systematically read for each analysis, referred to as "reliable markers". These markers are of special importance when one wishes to work on results of genotyping obtained in independent genotyping experiments possibly obtained by different genotyping techniques.

Herein is also presented a solution that allows improvement of both the quality of the interpretation of raw data, and also, the identification of markers of good quality (i.e. markers that can reliably be used in various experiments, leading to reliable (correct) results) through the use of appropriate indicators.

It is proposed to act in two steps.

The first step is to make sure that a marker that is considered as being of good quality for an analysis is systematically used in further analysis. It is possible to set up the parameters of the software to achieve this goal.

The second step is to identify, as finely as possible, which markers are to be systematically kept and used (reliable markers). To achieve this end, new indicators have been designed, which can be used with indicators already proposed in the literature (Affymetrix, Axiom Genotyping Solution Data Analysis Guide 702961 Rev. 1).

These indicators measure the reliability of the reading, marker by marker. They are mainly used during the first analyses when using a new batch of markers.

It is during these first analyses that the list of usable and reliable markers is determined and is permanently fixed, as a calibration phase.

The indicators as described below make possible to identify reliable markers. They are based on calculations of distances, densities and make it possible to analyze the position of the clusters associated with each of the alleles. The more the allelic groups are dense and remote from the others, the stronger is the level of confidence in the marker and the more reliable the marker is. Number of genotyped individuals for which no allele can be assigned is negatively correlated with the quality of the marker.

The elements below shall be taken into consideration when deciding whether a marker is of quality and reliable across multiple genotype runs:

- Each individual in a given cluster is significantly distant from the closest cluster.
- The medoids of each cluster are significantly distant from each other.
- Each individual in a given cluster is closer to individuals of this given cluster than to individuals of other clusters.

The following indicators can be calculated:

Table 1 : list of various indicators

| | |
|---|---|
| DistanceABAA | Distance between clusters AB and AA (distance between the Northing (value on Y axis) of the medoid of cluster AB and the Northing of the medoid of cluster AA) |
| DistanceABBB | Distance between clusters AB and BB (distance between the Northing of the medoid of cluster AB and the Northing of the medoid of cluster BB) |
| nAANA | Number of individuals not assigned between clusters AA and AB |
| nBBNA | Number of individuals not assigned between clusters BB and AB |
| maxAANA | Maximal distance between non assigned individuals and closest cluster AA or AB |
| maxBBNA | Maximal distance between non assigned individuals and closest cluster BB or AB |
| varAANA | Variance of values on the Y axis for non-assigned individuals located between clusters AA and AB |
| varBBNA | Variance of values on the Y axis for non-assigned individuals located between clusters BB and AB |
| DensityAA | Concentration of missing values (non-assigned individuals) between cluster AA and AB |
| DensityBB | Concentration of missing values (non-assigned individuals) between cluster BB and AB |
| MaxDensity | Max(DensityAA,DensityBB) which outputs the value that is the maximal one between the two input values |
| Distance ABAB | Distance between the two principal medoids of cluster AB |
| B.heterobelowHomo | Presence/absence of a heterozygous cluster below the two homozygous clusters (Y axis) |
| N.ABmin | If presence of two heterozygous clusters, minimal number of individuals between these two clusters |
| MinHomo | Lowest position (on the Y axis) of individuals of homozygous clusters (AA et BB) |
| MinAB | Position of the lowest médoid of the AB clusters on the Y axis |
| N.NA | Number of missing values (non-assigned alleles) |
| N.NAbelowHomo | Number of missing values (non-assigned alleles) under the homozygous clusters |
| *dunn* | *minimum separation / maximum diameter. Dunn index, see Halkidi et al. (2002)* |
| *wb. ratio* | *average. within/average. between* |
| Repro Intra | Use of the same DNA twice during the same run, and check that obtained alleles are the same. "HomDiff" difference is used when the same DNA is read as AA and as BB for the same marker. "HetDiff" difference is used when the same DNA is read as AA and as AB, or as BB and as AB for the same marker. |
| Repro Inter | Use of the same DNA twice during two different runs, and check that obtained alleles are the same. HomDiff and HetDiff are also used for this analysis |

The above indicators are not described in the art, apart from the *dunn* and the *wb.ratio.*
Indicators linked to the density of missing data between the clusters (DensityAA and DensityBB) are powerful quality indicators for a marker.
Similarly, indicators of reproducibility and repeatability (Repro Intra and Repro Inter) are powerful indicators to quickly identify problems related to specific markers and to permanently exclude these non-reliable markers.
Appropriate thresholds for *Densities, Repro* and *dunn* indicators make it possible to be very specific when sorting the markers to keep and the ones to discard.

It is preferred to calculate the above indicators with a sufficient number of samples, (i.e. preferably more than 30 samples) for each marker.

If Repro.Intra.Diff > 5 or Repro.inter.Diff>5) (Repro.Intra and Repro.Inter), it is considered that the markers are not reliable and they shall thus be removed from future consideration.

Markers are considered as reliable and will thus be retained for future analysis when these markers can be sorted in one of the following classes:

(a) Call rate below threshold (SNP call rate is below threshold, but other cluster properties are good)

(1) MaxDensity<30 OR
(2) 30<=MaxDensity<150 and Dunn>0.03

OR
(b) PAV (presence Absence Variant)

(1) one cluster AA, AB or BB does not exist
(2) OR no missing data between cluster and dunn>0.1
(3) OR MaxDensity <80

OR
(c) Other

(1) MaxDensity<30, OR
(2) 30<=MaxDensity<150 and dunn>0.03 and Call Rate >95

OR
(d) Poly High resolution
dunn>0.1 and MaxDensity <80

The invention thus also relates to a method for selecting markers that can be used for genotyping individuals, comprising the steps of

(a) inputting raw data (such as microarray data) obtained from a reference panel within a computer software for analyzing microarray genotyping data, wherein said computer software calculates, for each marker, clusters, wherein each individual is assigned to a cluster for each marker
(b) for each marker, calculating indicators that represent the adequacy of the data proposed by said computer software and the expected data
(c) Selecting a marker for future use for genotyping individuals if the indicators are above a predetermined threshold

In particular, the method uses at least one, and preferably all of the following indicators:

- HomDiff_loc = for each loci, the percentage of individuals for which the allele determined is homozygous and the expected allele is homozygous but inversed [with the other genotype [allele]]
- HetDiff_loc = for each loci, the percentage of individuals for which the allele determined is homozygous and the expected allele is hetereozygous or for which the allele determined is heterozygous and the expected allele is homozygous

Markers are discarded (i.e. the markers are not taken into consideration or used in analysis of future runs) when the threshold for HomDiff loc is above 5.

Also disclosed but not part of the invention is a non-transitory computer readable storage medium having stored thereon processor-executable software instructions configured to cause a processor of a computing device to perform operations comprising: for each marker used in a genotyping program, calculating indicators that represent the adequacy of the data proposed by the genotyping software and the expected data, and optionally providing a signal when the result of the indicators are below a predetermined threshold.

It is to be noted that it is possible to repeat the steps of generating the reference panel, improving the settings of the computer software and optimizing the marker set. Indeed, when the marker set has been optimized with a given reference panel (an given settings), it is possible to set-up a new reference panel, using this marker set, and implement again the steps of determining proper settings for the computer program and assessing the marker quality as described above. This can be repeated a few times, in order to obtain strong reference panel, settings and marker set.

**Presence / Absence of the allele (PAV markers)**

Correcting the reading of markers of PAV (Presence / Absence of variant) is already present in commercial softwares (such as the ones commercialized by Affimetrix).

However many markers of PAV type are not detected and an individual for which there is absence of the allele in a PAV marker is frequently interpreted incorrectly as an individual bearing a heterozygous allele.

Optimization of setting such that been mentioned above favorably contributes to detection of markers of PAV type

The optimization of the choice of indicators allows to significantly improve the detection of these markers and thus to allocate a 4th allele (PAV / absence of signal / no allele) to as many relevant markers as possible.

This is performed by an analysis of the heterozygous cluster. Briefly, it is checked whether it makes sense to split the heterozygous cluster into two heterozygous clusters (one that would be contain individual being actually heterozygous, and the other that would contain individuals that don't have a signal (absence of signal)).

In particular, the distance between these two sub-clusters/subgroups (Distance ABAB) is calculated, and the marker can be classified as PAV if this distance is significant (higher than a given threshold).

It also checked whether the missing data (i.e. the individuals for which no allele has been assigned for this marker) can also be considered as included within a fourth cluster corresponding to "absence of the allele". It is to be noted that one shall not automatically consider that any missing data has to be considered as representing an "absence of the allele", but that it also necessitates to verify whether the missing data can be regrouped in a cluster which makes sense from a genetics point of view. These verifications are made by looking at the position of the missing data, with each other and with respect to the AA, AB and BB clusters.

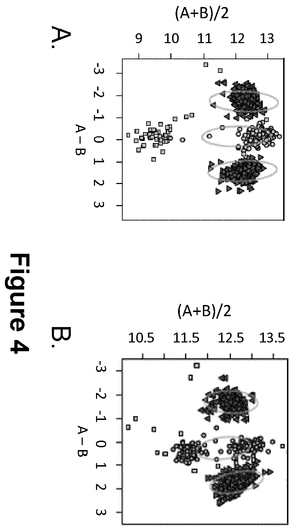

For the markers as depicted in Figure 4, the indicators used in the existing routines don't detect the fourth cluster.

Indeed, in Figure 4.A the fourth cluster detection is generally done through the use of an indicator called HetSO (Heterozygous Cluster Strength Offset). This indicator measures the distance in the vertical direction between the center of the Hetero cluster and the center of the Homo cluster. However, this doesn't take into account the presence of a large number of missing data under the Heterozygote cluster.

In figure 4.B, the HetSo indicator does not make it possible either to detect the fourth cluster because the center of the cluster on the Y axis ($(A+B)/2$) is not sufficiently distant from the center of the homozygous cluster.

The consequences of the misclassification of these types of marker is that a large number of data, that should have been considered as the absence of the allele, will not be analyzed, thus reducing the quantity and quality of information obtained from the experiment.

Furthermore, this would lead to erroneous conclusions at the time where this marker is used in analysis routines.

In order to properly classify a marker as a PAV, or in other terms, to determine whether one should assign a cluster "Absence of signal" to a marker, the heterozygous cluster will artificially be split into two new clusters of individuals.

The principle is to test whether the heterozygous cluster that is indicated to include heterozygous individuals (i.e. individuals that have not been assigned to a homozygous cluster) can actually regroup two clusters (one that contain the heterozygous individuals, i.e. individuals containing the two versions of the allele, and the other that would contain individuals that don't bear any version of the allele detected by the marker (absence of the signal).

Various tests will be made on the two clusters (using, in particular, the coordinates of the medoid of each cluster as representative of the cluster) in order to check which hypothesis makes the more sense:

- Actual existence of two clusters (heterozygous and "absence of a signal" clusters), whereas the marker can then be classified as PAV
- Existence of only one cluster (heterozygous cluster)

The invention thus relates to a method for determining whether a cluster "Absence of signal" is to be assigned to a marker used in a genotyping experience comprising the steps of

(i) inputting raw data (such as the data obtained directly from the microarray) obtained from multiple individuals within a computer software for analyzing microarray genotyping data, wherein said computer software calculates, for each marker, clusters, wherein each individual is assigned to a cluster for each marker or may be assigned to a cluster for each marker.

(ii) for each marker,

(b1) artificially separating the heterozygous cluster AB in two new clusters, so as to obtain two new clusters and two medoids (one for each cluster medoid1 and medoid2)

Verifying conditions (1) to (4) below

Condition (1)

(a1) calculating the value *HomMin* = min($q_{5\%}(Y_{AA})$,$q_{5\%}(Y_{BB})$), with $q_{5\%}(Y_{AA})$ being the quartile at 5% of all $Y_i$ of cluster AA ($Y_i$ being the value of individual i on the Y axis), and $q_{5\%}(Y_{BB})$ being the quartile at 5% of all $Y_i$ of cluster BB ($Y_i$ being the value of individual i on the Y axis),
(c1) calculating the distance between the medoid having the lowest Northing (Y value) and the HomMin value
(d1) calculating the value Dist.ABAB= (Ymedoid1 - Ymedoid2) with Ymedoid1 being the Northing of the highest medoid (value on the Y axis) and Ymedoid2 being the Northing of the lowest medoid (value on the Y axis).
(e1) calculating N.ABmin, corresponding to the lowest number of individuals in the two new heterozygous clusters

Wherein a cluster "Absence of signal" is assigned to the marker if the value calculated in (c1) is higher than a first predetermined threshold (preferably 0.2), and the value calculated in (d1) higher than a first predetermined threshold (preferably 0.4) and the value calculated in (e1) higher than a first predetermined threshold (preferably 5),
And
Condition (2)
(a2) calculating N.NAbelowHomo : number of individuals for which no allele has been assigned, and having a Northing (Y value) below HomMin, and an Easting between the Easting (X value) of the medoid of cluster AA and the easting of the medoid of cluster BB
Wherein a cluster "Absence of signal" is assigned to the marker if the value calculated in (a1) is higher than a first predetermined threshold (preferably 8)
And
Condition (3)

(a3) if
a3i) the Northing of a medoid of one of the heterozygous cluster is below the lowest Northing of the medoid of the AA or BB clusters, with the distance in Northing between above a predetermined threshold (preferably 0.2), and
a3ii) the distance between the two heterozygous clusters (Dist.ABAB) is above a predetermined threshold (preferably 0.4), and
a3iii) the number of individuals

- in the lowest heterozygous cluster and
- which have been assigned to no cluster

is above a predetermined threshold (preferably 5),

wherein a cluster "Absence of signal" is assigned to the marker is all conditions a3i to a3iii are fulfilled
And
Condition (4)
(a4) Calculating the value HomMin - Min(Ymedoid1,Ymedoid2) - Dist.ABAB

Wherein a cluster "Absence of signal" is assigned to the marker if the value calculated in (a4) is higher than a first predetermined threshold (preferably 0.2) and the number of individuals in the lowest heterozygous cluster is higher than a first predetermined threshold (preferably 5)
(iii) Assigning a cluster "Absence of signal" to the marker if at least one of the conditions (1) to (4) is fulfilled.

Some individuals can't be assigned to a cluster AA, AB or BB and would thus be assigned to a "virtual" cluster called MISSING. If there are at least 5 individuals in this MISSING cluster, and the MISSING cluster is under the AB cluster, this MISSING cluster is postentially a PAV cluster and the method above should merge this MISSING cluster and the new cluster "Absence of signal" created.
As already indicated above, computer softwares used to perform step (i) of this method are known in the art and commercialized by various manufacturers, such as Affymetrix of Illumina.
In step (ii) (b1), the separation of the original heterozygous cluster in two new clusters is performed by using any software or statistical function in the art such as the pam function available on the R software or by partitioning the data into 2 clusters around medoids, with the method as described in Reynolds, A., Richards, G., de la Iglesia, B. and Rayward-Smith, V. (1992) Clustering rules: A comparison of partitioning and hierarchical clustering algorithms; Journal of Mathematical Modelling and Algorithms 5, 475-504. This partition is performed with the statistical software known in the art, determining, for each individual of the heterozygous cluster, whether it should be assigned to one or the other cluster, from a statistical point of view. Eventually, all individuals present in the original heterozygous cluster are assigned to one of the newly created cluster.

Each new cluster possesses a medoid, the mathematically representative object in the cluster of individuals, which has the smallest average dissimilarity to all other individuals in the cluster. The coordinates of the medoid for the clusters can be calculated by the software that created the new clusters.

The last (but that could be optional) step of the method is to assign the qualification "absence of a signal" to the marker if at least one of the conditions (1) to (4) is fulfilled. Consequently, an individual shall be assigned to four clusters for this marker (Homozygous AA, Homozygous BB, Heterozygous AB or Absence of signal) rather than to the three clusters AA, BB and AB for other markers.

Also disclosed but not part of the invention is a non-transitory computer readable storage medium having stored thereon processor-executable software instructions configured to cause a processor of a computing device to perform operations comprising:

- receiving raw data obtained from multiple individuals
- calculating, for each marker, clusters, wherein each individual is assigned to a cluster for each marker or may be assigned to a cluster for each marker
- for each marker, artificially separating the heterozygous cluster AB in two new clusters, so as to obtain two new clusters and two medoids (one for each cluster medoid1 and medoid2)
- Verifying conditions (1) to (4) as indicated above
- Optionally assigning a cluster "Absence of signal" to the marker if at least one of the conditions (1) to (4) is fulfilled and recording the localization of said cluster.

The methods described above make it possible to improve the quality of the marker set, that it

(a) to parametrize the software analyzing the raw data by using a pre-determined set of data,
(b) to eliminate markers of wrong quality and
(c) to determine whether characteristic "absence of signal" shall be assign to a given marker.

These steps, either individually or in any combination will improve the quality of the reading and hence of the genotyping run.

**Quality of the allelic reading**

It is also interesting to improve the quality of the eventual allelic result that can be represented as a matrix of the form depicted in Table 1.

Table 2. Matrix representation of allelic data obtained after a genotype run (Ni: number of markers; Nj: number of samples/individuals)

| MarkerName | Sample 1 | Sample 2 | Sample Nj |
|---|---|---|---|
| Marker 1 | AA | BB | AB |
| Marker 2 | AB | BB | AB |
| ... | ... | | ... |
| Marker Ni | BB | AA | AB |

From this allelic information, it is possible to pose a diagnosis on the analysis process as a whole, and to respond to a few questions that are of importance to determine the reliability of the end result.

- Does the genotyped material correspond to the expected material? Was there any mix of material before DNA extraction?
- Were there some technical or operating problems during the process (such as error in sample position on the plate or plate inversions). Two plate inversion can exist : The read plate is not the expected one, because of a mix of plate or all the samples on the plate are on a wrong position due to distribution robot programming error and this specific situation some plate may have an inverted distribution.

The applicant used new indicators as disclosed below to perform both a diagnosis of "errors in material" (genotype material does not match the expected one) and of "technical errors" (existence of a problem during the technical process, which translates to one or more errors on several individuals).

Indicators will be calculated, marker by marker, in order to determinate the reliability of each marker (CallRate, marker class, HetRate, reproducibility).

Then indicators are calculated for the individual on a set of reliable markers (subset of the marker set chosen among the PolyHighResolution markers and the markers which have reproducibility indicators (Repro Intra or Repro Inter) below 1). It is also preferred when these markers are evenly distributed on the zone that is mapped for the individuals.

Using these indicators make it possible to have a good diagnosis on the quality of the run.

High-throughput genotyping as envisaged is performed on laboratory plates, generally 384-well lab-plates, that are known and widely used in the art.

Each well of a plate contains the DNA to analyze and the components required to perform this analysis (primers, labels to be able make the reading of the markers on the genotyping chip/array.

A genotyping run is generally performed for a large number of individuals (a few dozens or hundred), this will involve the use of a proportional number of plates. Using the the Affymetrix technology, plates containing 384 well are generally used, making it possible to test 384 DNA extracts from 384 starting samples. Generally, the DNA was extracted from the individuals using 96-well plates, and the 384 plate that will be used in the genotyping run corresponds to four 96 well plates put together (with a risk of error by plate inversion).

In the steps preceding the actual genotyping step, there may be different mistakes (such as inversion of plates or contamination of the material) that could potentially lead to inconsistent results of interpretation of the genotyping data (such as allocating allelic result to wrong individuals in case of inversion of a plate). It is thus essential to be able to detect this type of errors that correspond to technical errors.

The detection of these errors can be made by using two types of controls:
Negative controls such as empty wells where no signal should be read. A signal read in these wells may result from a contamination or plate inversion. These empty wells should have very low dQC and Call Rate (dQC lower than 0.7and ; CallRate lower than 92%). The position of these empty wells is an identifying element of the plates: the position of this negative control is different from one plate to another. Using this information makes it possible to determine which kind of inversion takes place.

Positive controls such as wells where the DNA comes from individuals for which the expected alleles are known. Unlike the "panel of reference" disclosed above which was used to parametrize the genotyping software and for which only digital data is entered in the genotyping software, these positive controls correspond to physical samples that follow almost the same steps as other samples to genotype (it's added to the plates just after DNA extraction).In each 96-well plate, one can use two positive controls, such as a hybrid (control for heterozygosity) and a line (control of homozygosity). This couple is preferably unique for each plate of a program and can be used to identify the plate. If the allelic data obtained for these specific wells doesn't correspond to individuals placed in these wells on the plate but is found on another plate, then one can conclude that there was a substitution of these two plates.

It may be interesting to use indicators that are also linked to information of reference, using a reference Dataset that can be linked to the genotyping software. This reference Dataset shall contain information about individuals, and in particular genotyping information about these individuals. This genotyping information may be complete (for all the markers) or only for a limited number of markers. This genotyping information is preferably robust, in the meaning that it has been verified (in particular with indicators as herein described or by reproducibility).

This Dataset reference makes it possible to check and test the reproducibility intra and inter programs (see table 1), the consistence of results obtained for an individual present in multiple plates within a genotype experiment, or the consistence with results obtained for an individual in the dataset, that has already been genotyped in previous experiments.

It is also possible and very interesting to use the information from the dataset about pedigree of the individual. The pedigree corresponds to the genealogy information: parents (line, hybrids, population...) and breeding steps (cross, self-pollination, double haploids...) made to obtain the individual. Using pedigree information makes it possible to check for consistency between the declared pedigree (and thus the expected alleles) and the genotyping data (the observed alleles).

A new indicator Pedigree.ErrorRate was thus developed and is calculated for each individual.

$$Pedigree.ErrorRate = \text{(number of loci with impossible alleles) / (Number of loci where both parents are homozygous).}$$

If Pedigree.ErrorRate is higher than a defined threshold (5 is a preferred threshold), the individual is considered as not correct and a warning signal is emitted.

The invention thus relates to a method for genotyping individuals, wherein said genotyping has been performed on multiple individuals in a single run, wherein the parent genotype of some individuals in the run is known for at least some markers used for genotyping, comprising the steps of

a) allocating alleles to each individuals of the genotyping run
b) for each of the individuals for which parents genotype is known for at least some markers, calculating the value Pedigree.ErrorRate, wherein said value is

$$\text{Pedigree.ErrorRate} = (\text{number of loci with impossible alleles}) / (\text{Number of}$$
$$\text{loci where parents are homozygous})$$

c) emitting a signal (such as an alert) indicating that the results associated with the run are susceptible to be aberrant, if the value Pedigree.ErrorRate above is higher than a predetermined threshold.

In a preferred embodiment, other indicators are calculated.

In particular, the method may further comprising, in step b), the step of calculating parameters HomDiff_ind, HetDiff_ind, for each of said individuals for which parent genotype is known for at least some markers, wherein

i) HomDiff_ind = for each individual, the percentage of loci where the allele determined for said individual is homozygous and the expected allele is homozygous but inversed
ii) HetDiff_ind = for each individual, the percentage of loci where the allele determined for said individual is homozygous and the expected allele is hetereozygous or the allele determined for said individual is heterozygous and the expected allele is homozygous, and

displaying a warning where an indicator HomDiff_ind or HetDiff_ind are above a pre-determined threshold (preferably 5).

As an illustration, if the individual is identified as AA while its 2 parents are BB, this locus is considered a locus with impossible alleles. If one of the parent is AB and the other BB, the allele AA for an F1 progeny can be possible only if the cross between parents is followed by an haplo diploidisation step.

It is reminded that the identification of the parents of an F1 seed, can currently be done by pericarp genotyping, and that the methods as disclosed herein, alone or in any combination, can also be applied to F1 seeds of an A and B inbred line, possibly preexisting, and the parent profile inferred with a combination of statistical and genotyping methods, leveraging in particular other available F1 using A, B and other inbred lines.

Imputation process can also be a part of the genotyping process: using high density data to impute from medium to low density data.

The imputation process can leverage such pipeline using some common indicators and validation process.

The methods as disclosed herein, alone or in any combination, can also be combined with methods of automatic allele calling. Based on statistical models and genetic knowledge, these methods enable to get the right alleles for any kind of material (seeds, leafs, bulks of seeds...) and for any kind of set of markers (low plex, midplex, multiplex) and any class of marker (high, medium and low quality).

The methods as disclosed herein, alone or in any combination, can also be combined with methods of statistical process control (SPC) to further improve the quality of the generated data. This type of tools enables detection of possible drifts over time in the genotyping process: from the reception of seeds to data import in databases. The concept is based on charts and indicators flagging irregular tendencies and enabling to detect where irregularities come from (equipment, raw material, temperatures and so on).

## Description of the Figures

Figure 1: Schematic representation of the various steps for performing a preferred embodiment of the method of genotyping.

Figure 2: Illustration of the representation of clusters with a computer software Axiom Analysis Suite Version 1.1. used to genotype individuals for a specific marker. The two homozygous clusters are on each side of the heterozygous cluster in the center of the representation. Each dot represents a genotyped individual. Contrast = Log2(A_signal/B_signal); Size = [Log2(A_signal) + Log2(B_signal)]/2.

Figure 3: analysis of a marker and assignment of the status "PAV" (Off-Target Variant) to this marker, using scripts provided by Affymetrix. On can see the presence of four clusters, including an "Absence of marker" (OTV) cluster located on the center at the bottom of the figure, below the heterozygous cluster.

Figure 4: examples of markers that are not detected as PAV markers using routines and indicators of the art.
Figure 5: algorithm of decision for quality control of plates reading.

## Examples

## Example 1 - computer assisted process for performing a genotyping run.

All the methods described above can be implemented with a single tool (a computer software).
In this case, the user would input a setting/parameter data file (the one that was obtained with the reference panel), and launch the executable software.
When the analysis is complete, a report can be published and can be checked for any action to be made (such as discarding plates or the like if one of the various controls on the plates shows that there is a problem) before using the generated allelic data.
Thus, one will use a system that will use the following to deliver genotyping information:

- a file directory containing the raw data (such as files with the extension ".CEL out of Affymetrix chips);
- a file listing the samples to genotype and containing, in particular, the position of the samples on the plates, their unique identifier and any other information (such as information on the samples pedigree) that can be used to ensure the finest quality control;
- a.bat file or the like in order to launch the analysis, comprising all of the algorithms described above and linked to the quality analysis

To run the analysis software (analysis of the raw data obtained from the chip in order to allocate alleles to individuals), one will launch the file with the .bat extension.
Once the analysis is completed (all checks have been performed, and allele information has been allocated for as many markers and individuals), the user shall receive a notification informing of the end of the analysis, and a log file can be created. This log file makes it possible to check that the various audit were properly conducted and that all steps have taken place. Finally, a report would summarize all of the conclusions and output data.
Output files from the report would allow a more detailed analysis of the analyzed material. They would include all of the indicators for the controls, markers, plates and samples. They include also all the distance calculations, reproducibility information and verification of pedigree.

## Example 2. Using the reference panel

The impact of using the panel of reference to determine the right settings that will be used and help classify the markers can be evaluated by observing the difference in the number of polyhighResolution markers (i.e. markers of good quality) with or without using the panel. The relative amount of such markers is a representative indicator of the quality of the genotyping data obtained with these softwares.
For a genotyping experiment on 68 individuals, it was observed that the number of polyhighResolution markers increased from 40% to 80% when the *reference panel* of 384 individuals was used for setting-up the software.

### Program of genotyping corn including 1046 individuals

This is also confirmed in a genotyping program genotyping including 1046 individuals. The reference panel of 384 individuals used is the same as above. The polyhighResolution markers increased from 43% to 76%.
Another way to check the impact of the use of this reference panel on the genotyping data quality is to look at reproducibility and repeatability of the results.
One can check whether the level of consistence of the output obtained with or without using the reference panel with the expected reference data.
In the case of the program based on 1046 individuals above, it was noted that the fact of using the reference panel decreases the number of samples with problems of reproducibility.

## Example 3: Optimization of the number of reliable markers:

The tools implemented to optimize the number of reliable markers and determine the set of operational markers that can be used in a genotyping program allowed to increase the number of markers used in routine

| Plant | Percentage of markers used before optimization[1] | Percentage of markers used after optimization |
|---|---|---|
| Maize | 77 | 78 |
| Sunflower | 66 | 92 |
| Barley | 48 | 72 |
| Wheat | 18 | 83 |
| Rape | 37 | 73 |

These estimations are the ones communicated by the chip provider at the very first stage; it is highly correlated to the set of individuals

**Example 4: Correction of the interpretation:**

The use of additional indicators for the detection of the PAV made it possible to correct on average 5% of the markers and thus correct the alleles attributed to these markers for about 2% of individuals.

**Example 5: control of the consistency of the results:**

Introduction and use of the new pedigree indicator, as well as the various other controls, made it possible to detect 2 plate inversions in genotyping programs with of more than 1000 individuals.

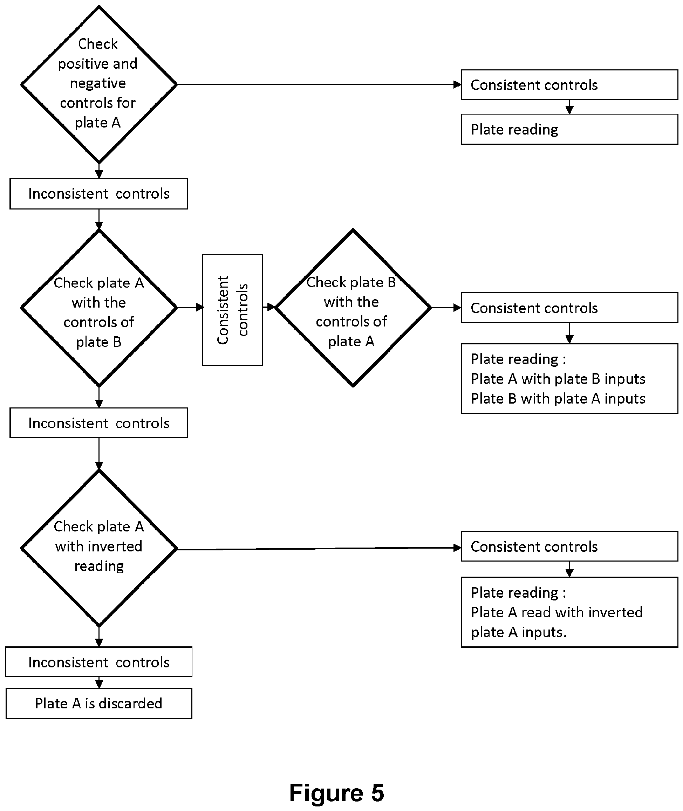

A specific plate inversion protocol may be included in a software with the following steps, as disclosed in Figure 5: If some positive or negative controls are not consistent for plate A, this plate is provisionally classified as failed and other verifications need to be done (Figure 5).

Step 1, if it is possible to find a plate B for which the controls results are the one expected for plate A (and vice-versa), the two plates are considered as having been inverted and rectification is made.

If this is not possible, a second step of verification shall be done. Plate A is read as if it had been inverted and if the new reading is consistent with the expected reading, the plate A is kept and interpreted accordingly.

If controls for plate A are still inconsistent after this second step, the run is considered as failed and the plate is discarded.

This control makes it possible to detect these inversions and not to discard or misread the plates. With this control, these plates can be retreated and the data used with the implementation of the method.

This process also made it possible to identify other types of errors such as material error, or error in declaration of the nature of the material genotyped..., giving the user the opportunity to correct.

**Claims**

1.  A computer-implemented method for optimizing the settings of a computer program that analyzes raw data of a genotyping run by assigning each individual of the genotyping run to a genotype cluster, thereby assigning allelic information for such individuals, by:

    (a) introducing raw data from individuals of a reference panel in said computer software wherein the allele information of these individuals is known,
    (b) introducing the allele information for individuals of this reference panel in said computer software.
    (c) varying the settings of the computer program to determine those that provide the best output data, as compared to the data known for the individuals of the reference panel, wherein the output data is the locations where the allele clusters are expected for the considered markers.

2.  The method of claim 1, further comprising recording of the optimized setting of i.(c) that provide the best output data.

3.  The method of claim 2, wherein the recorded optimized settings comprise the equations of the ellipses that correspond to each allele class for each marker.

4.  The method of any one of claims 1 or 2, wherein said individuals in said reference panel have been chosen according to a method comprising the steps of:

i. Determining a minimal number, n, of individuals that need to be present in each genotype cluster
ii. Selecting n random individuals from a starting panel for which the allelic information is known for each individual
iii. Adding one new individual from said starting panel, wherein said added individual is chosen so as to most increase the number of genotype clusters that respond to the desired condition (at least n individuals per genotype cluster)
iv. Repeating step (c) until at least n individuals per genotype cluster are obtained.

5. The method according to claim 4, wherein the individuals of the reference panel have genotyped more than once with always the same result or are the progeny of parents for which the genotype is known.

6. The method according to claim 4 or 5, wherein the minimal number n of individuals that need to be present in each genotype cluster is 5.

7. The method of any one of claims 1 to 6, further comprising selecting markers that can be used for genotyping individuals by:

   (a) inputting raw data obtained within a computer program for analyzing microarray genotyping data, wherein said computer programs calculates, for each marker used during the run, clusters, wherein each individual is assigned to a cluster for each marker
   (b) for each marker, calculating indicators representing the reliability of the marker,
   wherein a marker is selected for future use for genotyping individuals if the indicators are above a predetermined threshold, and
   wherein said indicators are

      (a) HomDiff_loc = for each locus, the percentage of individuals for which the allele determined is homozygous and the expected allele is homozygous but inversed [with the other genotype [allele]]
      (b) HetDiff_loc = for each locus, the percentage of individuals for which the allele determined is homozygous and the expected allele is hetereozygous or for which the allele determined is heterozygous and the expected allele is homozygous

8. The method of claim 7, wherein said indicators are *DensityAA* corresponding to the concentration of missing values between homologous cluster "AA" and heterologous cluster "AB" or *DensityBB* corresponding to the concentration of missing values between homologous cluster "BB" and heterologous cluster "AB".

9. The method of any one of claims 7 to 8, wherein the markers that can be sorted in one of the following classes are considered as reliable if:

   (a)

      (1) MaxDensity<30 OR
      (2) 30<=MaxDensity<150 and Dunn>0.03

   OR
   (b)

      (1) one cluster AA, AB or BB does not exist
      (2) OR no missing data between cluster and dunn>0.1
      (3) OR MaxDensity <80

   OR
   (c)

      (1) MaxDensity<30, OR
      (2) 30<=MaxDensity<150 and dunn>0.03 and Call Rate >95

   OR
   (d)
   dunn>0.1 and MaxDensity <80.

10. The method of any one of claims 1 to 9, wherein a cluster "Absence of signal" has been assigned to a marker used in the genotyping run, wherein said cluster has been assigned by a method comprising the steps of

(i) inputting raw data obtained from multiple individuals within a computer program for analyzing microarray genotyping data, wherein said computer programs calculates, for each marker, genotype clusters, wherein each individual is assigned to a cluster for each marker
(ii) for each marker,
(b1) separating the heterozygous cluster AB in two new heterozygous clusters, so as to obtain two new clusters and two medoids (one for each cluster)

Condition (1)

(a1) calculating the value HomMin=min($q_{5\%}(Y_{AA})$,$q_{5\%}(Y_{BB})$), with $q_{5\%}(Y_{AA})$ being the quartile at 5% of all Yi of cluster AA (Yi being the value of individual i on the Y axis), and $q_{5\%}(Y_{BB})$ being the quartile at 5% of all Yi of cluster BB (Yi being the value of individual i on the Y axis),
(c1) calculating the distance between the medoid having the lowest Northing and the HomMin value
(d1) calculating the value Dist.ABAB= Abs ($Y_{medoïd1}$ - $Y_{medoïd2}$) with $Y_{medoid1}$ being the Northing of the highest medoid (value on the Y axis) and $Y_{medoid2}$ being the Northing of the lowest medoid (value on the Y axis).
(e1) calculating N.ABmin, corresponding to the lowest number of individuals in the two new heterozygous clusters

Wherein a cluster "Absence of signal" is assigned to the marker if the value calculated in (c1) is higher than a first predetermined threshold, and the value calculated in (d1) higher than a first predetermined threshold and the value calculated in (e1) higher than a first predetermined threshold,
And
Condition (2)

(a2) calculating N.NAbelowHomo : number of individuals for which no allele has been assigned, and having a Northing below HomMin, and an Easting between the Easting of the medoid of cluster AA and the easting of the medoid of cluster BB
Wherein a cluster "Absence of signal" is assigned to the marker if the value calculated in (a2) is higher than a first predetermined threshold
And

Condition (3)

(a3) if
a3i) the Northing of a medoid of one of the heterozygous cluster is below the lowest Northing of the medoid of the AA or BB clusters, with the distance in Northing being above a predetermined threshold, and
a3ii) the distance between the two heterozygous clusters (Dist.ABAB) is above a predetermined threshold, and
a3iii) the number of individuals in the lowest heterozygous cluster and which have been assigned to no cluster is above a predetermined threshold, wherein a cluster "Absence of signal" is assigned to the marker is all conditions a3i to a3iii are fulfilled

And
Condition (4)
(a4) Calculating the value HomMin - Min($Y_{medoid1}$,$Y_{medoid2}$ ) - Dist.ABAB
Wherein a cluster "Absence of signal" is assigned to the marker if the value calculated in (a4) is higher than a first predetermined threshold and the number of individuals in the lowest heterozygous cluster is higher than a first predetermined threshold

(iii) Assigning a a cluster "Absence of signal" to the marker if at least one of the conditions (1) to (4) is fulfilled.

11. The method of claim 10, wherein the thresholds are

Condition 1:

0.2 for the value calculated in (c1)
0.4 for the value calculated in (d1)
5 for the value calculated in (e1)

Condition 2:
8 for the value calculated in (a2)
Condition 3

0.2 for the distance in Northing
0.4 for the distance between the two heterozygous clusters
5 for the number of individuals in the lowest heterozygous cluster and which have been assigned to no cluster

Condition 4

0.2 for the value calculated in (a4) and
5 for the number of individuals in the lowest heterozygous cluster

**12.** A computer program product, that analyze raw data of a genotyping run, comprising instructions to assign each individual of a genotyping run to a genotype cluster, when such instructions are executed by a logical circuit or a processor, comprising settings optimized by a method according to any one of claims 1 to 11.

**13.** A computer-implemented method for genotyping individuals of a genotyping run, comprising the steps of

i. providing raw data from the individuals to a computer program product according to claim 12,
ii. allocating alleles to each individuals of the genotyping run
iii. recovering the genotyping information for each individual of the genotyping run.

**14.** The method of claim 13, wherein raw data from individual of the reference panel used for setting optimization of the computer program is provided together with the raw data from the individuals of the genotyping run.

**15.** The method of claim 14, wherein a signal of the poor quality of raw data of the genotyping run is detected if the only clear assignation of alleles is obtained for the data from the reference panel.

**16.** A computer device, comprising a logical circuit, comprising settings optimized by the method according to any one of claims 1 to 11 and executing instructions to assign each individual of a genotyping run to a genotype cluster, connected to a human/machine interface device so as to perform the method according to any of the claims 13 to 15.

**Patentansprüche**

**1.** Computerimplementiertes Verfahren zur Optimierung der Einstellungen eines Computerprogramms, das Rohdaten eines Genotypisierungslaufs analysiert, indem es jedes Individuum des Genotypisierungslaufs einem Genotyp-Cluster zuordnet, wodurch allelische Informationen für solche Individuen zugewiesen werden, indem:

(a) Einführung von Rohdaten von Individuen eines Referenzpanels in die Computersoftware, wobei die Allelinformationen dieser Individuen bekannt sind,
(b) Einführung der Allelinformationen für Individuen dieses Referenzpanels in die Computersoftware.
(c) Variieren der Einstellungen des Computerprogramms, um diejenigen zu bestimmen, die im Vergleich zu den für die Individuen des Referenzpanels bekannten Daten die besten Ausgabedaten liefern, wobei die Ausgabedaten die Orte sind, an denen die Allelcluster für die betrachteten Marker erwartet werden.

**2.** Verfahren nach Anspruch 1, das ferner die Aufzeichnung der optimierten Einstellung von i.(c) umfasst, die die besten Ausgangsdaten liefert.

**3.** Verfahren nach Anspruch 2, wobei die aufgezeichneten optimierten Einstellungen die Gleichungen der Ellipsen umfassen, die jeder Allelklasse für jeden Marker entsprechen.

4. Verfahren nach einem der Ansprüche 1 oder 2, wobei die Individuen im Referenzpanel nach einem Verfahren ausgewählt wurden, das die folgenden Schritte umfasst:

i. Bestimmung der Mindestanzahl n von Individuen, die in jedem Genotyp-Cluster vorhanden sein müssen

ii. Auswahl von n zufälligen Individuen aus einem Ausgangspanel, für das die Allelinformation für jedes Individuum bekannt ist

iii. Hinzufügen eines neuen Individuums aus dem Ausgangspanel, wobei das hinzugefügte Individuum so ausgewählt wird, dass es die Anzahl der Genotyp-Cluster, die auf die gewünschte Bedingung reagieren, am meisten erhöht (mindestens n Individuen pro Genotyp-Cluster)

iv. Wiederholung von Schritt (c), bis mindestens n Individuen pro Genotyp-Cluster erhalten werden.

5. Verfahren nach Anspruch 4, wobei die Individuen des Referenzpanels mehr als einmal mit stets demselben Ergebnis genotypisiert wurden oder die Nachkommenschaft von Eltern sind, deren Genotyp bekannt ist.

6. Verfahren nach Anspruch 4 oder 5, wobei die Mindestanzahl n von Individuen, die in jedem Genotyp-Cluster vorhanden sein müssen, 5 beträgt.

7. Verfahren nach einem der Ansprüche 1 bis 6 umfasst ferner die Auswahl von Markern, die für die Genotypisierung von Individuen verwendet werden können, durch:

(a) Eingabe der erhaltenen Rohdaten in ein Computerprogramm zur Analyse von Microarray-Genotypisierungsdaten, wobei das Computerprogramm für jeden während des Laufs verwendeten Marker Cluster berechnet, wobei jedes Individuum für jeden Marker einem Cluster zugeordnet wird

(b) für jeden Marker, wobei Indikatoren für die Zuverlässigkeit des Markers berechnet werden,

wobei ein Marker für die zukünftige Verwendung zur Genotypisierung von Individuen ausgewählt wird, wenn die Indikatoren über einem vorbestimmten Schwellenwert liegen, und wobei die Indikatoren sind

(a) HomDiff loc = für jeden Locus der Prozentsatz der Individuen, bei denen das ermittelte Allel homozygot ist und das erwartete Allel homozygot, aber invers [mit dem anderen Genotyp [Allel]] ist

(b) HetDiff_loc = für jeden Locus der Prozentsatz der Individuen, bei denen das ermittelte Allel homozygot und das erwartete Allel heterozygot ist oder bei denen das ermittelte Allel heterozygot und das erwartete Allel homozygot ist

8. Verfahren nach Anspruch 7, wobei die Indikatoren *DensityAA* entsprechend der Konzentration der fehlenden Werte zwischen dem homologen Cluster "AA" und dem heterologen Cluster "AB" oder *DensityBB* entsprechend der Konzentration der fehlenden Werte zwischen dem homologen Cluster "BB" und dem heterologen Cluster "AB" sind.

9. Verfahren nach einem der Ansprüche 7 bis 8, wobei die Marker, die in eine der folgenden Klassen eingeordnet werden können, als zuverlässig gelten, wenn:

(a)

(1) MaxDensity<30 ODER
(2) 30<=MaxDensity<150 und Dunn>0,03

Oder
(b)

(1) ein Cluster AA, AB oder BB ist nicht vorhanden
(2) OR keine fehlenden Daten zwischen Cluster und dunn>0,1
(3) OR MaxDensity <80

Oder
(c)

(1) MaxDensity<30, OR
(2) 30<=MaxDensity<150 und dunn>0,03 und Call Rate >95

Oder

(d)

dunn>0,1 und MaxDensity <80.

**10.** Verfahren nach einem der Ansprüche 1 bis 9, wobei einem im Genotypisierungslauf verwendeten Marker ein Cluster " Abwesenheit des Signals " zugewiesen wurde, wobei der Cluster durch ein Verfahren zugewiesen wurde, das die folgenden Schritte umfasst

(i) Eingeben von Rohdaten, die von mehreren Individuen erhalten wurden, in ein Computerprogramm zum Analysieren von Microarray-Genotypisierungsdaten, wobei das Computerprogramm für jeden Marker Genotyp-Cluster berechnet, wobei jedes Individuum einem Cluster für jeden Marker zugeordnet wird

(ii) für jeden Marker,

(b1) Aufteilung des heterozygoten Clusters AB in zwei neue heterozygote Cluster, um zwei neue Cluster und zwei Medoiden (eines für jeden Cluster) zu erhalten

Voraussetzung (1)

(a1) Berechnung des Wertes HomMin=min($q_{5\%}$ ($Y_{AA}$ ),$q_{5\%}$ ($Y_{BB}$ )), wobei $q_{5\%}$ ($Y_{AA}$ ) das Quartil bei 5% aller Yi des Clusters AA (Yi ist der Wert des Individuums i auf der Y-Achse) und $q_{5\%}$ ($Y_{BB}$ ) das Quartil bei 5% aller Yi des Clusters BB (Yi ist der Wert des Individuums i auf der Y-Achse) ist,

(c1) Berechnung des Abstands zwischen dem Medoid mit der niedrigsten Ordinate und dem HomMin-Wert

(d1) Berechnung des Wertes Dist.ABAB= Abs ($Y_{medoïd1}$ - $Y_{medoïd2}$), wobei $Y_{medoid1}$ die Ordinate des höchsten Medoids (Wert auf der Y-Achse) und $Y_{medoid2}$ die Ordinate des niedrigsten Medoids (Wert auf der Y-Achse) ist.

(e1) Berechnung von N.ABmin, entsprechend der geringsten Anzahl von Individuen in den beiden neuen heterozygoten Clustern

Wobei dem Marker ein Cluster " Abwesenheit des Signals " zugewiesen wird, wenn der in (c1) berechnete Wert höher als ein erster vorbestimmter Schwellenwert ist und der in (d1) berechnete Wert höher als ein erster vorbestimmter Schwellenwert und der in (e1) berechnete Wert höher als ein erster vorbestimmter Schwellenwert,

Und

Voraussetzung (2)

(a2) Berechnung von N.NAbelowHomo : Anzahl der Individuen, denen kein Allel zugeordnet wurde und die einen Ordinate unter HomMin und einen Abszisse zwischen dem Abszisse des Medoids von Cluster AA und dem Abszisse des Medoids von Cluster BB haben

wobei dem Marker ein Cluster " Abwesenheit des Signals " zugewiesen wird, wenn der in (a2) berechnete Wert höher als ein erster vorbestimmter Schwellenwert ist

Und

Voraussetzung (3)

(a3) wenn

a3i) die Ordinate eines Medoids eines der heterozygoten Cluster liegt unter der niedrigsten Ordinate des Medoids der AA- oder BB-Cluster, wobei der Abstand in der Orinate über einem vorgegebenen Schwellenwert liegt, und

a3ii) der Abstand zwischen den beiden heterozygoten Clustern (Dist.ABAB) liegt über einem vorbestimmten Schwellenwert, und

a3iii) die Anzahl der Individuen im niedrigsten heterozygoten Cluster, die keinem Cluster zugeordnet wurden, über einem vorgegebenen Schwellenwert liegt,

wobei dem Marker ein Cluster " Abwesenheit des Signals " zugewiesen wird, wenn alle Bedingungen a3i bis a3iii erfüllt sind

Und

Voraussetzung (4)

(a4) Berechnen des Wertes HomMin - Min($Y_{medoid1}$, $Y_{medoid2}$) - Dist.ABAB

wobei dem Marker ein Cluster " Abwesenheit des Signals " zugewiesen wird, wenn der in (a4) berechnete Wert höher als ein erster vorbestimmter Schwellenwert ist und die Anzahl der Individuen im niedrigsten heterozygoten Cluster höher als ein erster vorbestimmter Schwellenwert ist

(iii) Zuweisung eines Clusters "Abwesenheit des Signals" an den Marker, wenn mindestens eine der Bedingungen (1) bis (4) erfüllt ist.

11. Verfahren nach Anspruch 10, wobei die Schwellenwerte sind

Voraussetzung 1:

0,2 für den nach (c1) berechneten Wert
0,4 für den nach (d1) berechneten Wert
5 für den in (e1) berechneten Wert

Voraussetzung 2:
8 für den in (a2) berechneten Wert
Voraussetzung 3

0,2 für die Entfernung in Ordinaten
0,4 für den Abstand zwischen den beiden heterozygoten Clustern
5 für die Anzahl der Individuen im niedrigsten heterozygoten Cluster, die keinem Cluster zugeordnet wurden

Voraussetzung 4

0,2 für den nach (a4) berechneten Wert und
5 für die Anzahl der Individuen im niedrigsten heterozygoten Cluster

12. Computerprogrammprodukt, das Rohdaten eines Genotypisierungslaufs analysiert, mit Anweisungen, um jedes Individuum eines Genotypisierungslaufs einem Genotyp-Cluster zuzuordnen, wenn solche Anweisungen von einer logischen Schaltung oder einem Prozessor ausgeführt werden, mit Einstellungen, die durch ein Verfahren nach einem der Ansprüche 1 bis 11 optimiert sind.

13. Computer-implementiertes Verfahren zur Genotypisierung von Individuen eines Genotypisierungslaufs, das die folgenden Schritte umfasst

i. Bereitstellung von Rohdaten der Personen für ein Computerprogrammprodukt nach Anspruch 12,
ii. Zuweisung der Allele zu den einzelnen Individuen des Genotypisierungslaufs
iii. die Genotypisierungsinformationen für jedes Individuum des Genotypisierungslaufs wiederherstellen.

14. Verfahren nach Anspruch 13, wobei Rohdaten von Individuen des Referenzpanels, die für die Einstellungsoptimierung des Computerprogramms verwendet werden, zusammen mit den Rohdaten der Individuen des Genotypisierungslaufs bereitgestellt werden.

15. Verfahren nach Anspruch 14, wobei ein Signal für die schlechte Qualität der Rohdaten des Genotypisierungslaufs erkannt wird, wenn die einzige eindeutige Zuordnung von Allelen für die Daten aus dem Referenzpanel erhalten wird.

16. Computervorrichtung mit einer logischen Schaltung, die Einstellungen enthält, die durch das Verfahren nach einem der Ansprüche 1 bis 11 optimiert wurden, und die Befehle ausführt, um jedes Individuum eines Genotypisierungslaufs einem Genotyp-Cluster zuzuordnen, und die mit einer Mensch/Maschine-Schnittstellenvorrichtung verbunden ist, um das Verfahren nach einem der Ansprüche 13 bis 15 durchzuführen.

**Revendications**

1. Une méthode mise en oeuvre par ordinateur pour optimiser les paramètres d'un programme informatique qui analyse les données brutes d'une série de génotypage en assignant chaque individu de la série de génotypage à un cluster de génotypes, assignant ainsi des informations alléliques pour ces individus, en :

(a) introduisant des données brutes provenant d'individus d'un panel de référence dans ledit programme informatique, les informations sur les allèles de ces individus étant connues,
(b) en introduisant les informations sur les allèles des individus de ce panel de référence dans ledit programme informatique,

(c) en faisant varier les paramètres du programme informatique afin de déterminer ceux qui fournissent les meilleures données de sortie, par rapport aux données connues pour les individus du panel de référence, les données de sortie étant les emplacements où les clusters d'allèles sont attendus pour les marqueurs considérés.

**2.** Méthode selon la revendication 1, comprenant en outre l'enregistrement des paramètres optimisés de i.(c) qui fournissent les meilleures données de sortie.

**3.** Méthode selon la revendication 2, dans laquelle les paramètres optimisés enregistrés comprennent les équations des ellipses qui correspondent à chaque classe d'allèles pour chaque marqueur.

**4.** Méthode selon l'une quelconque des revendications 1 ou 2, dans laquelle les individus dudit panel de référence ont été choisis selon une méthode comprenant les étapes de

i. Déterminer un nombre minimal, n, d'individus qui doivent être présents dans chaque groupe de génotype
ii. Sélectionner n individus aléatoires à partir d'un panel de départ pour lequel les informations alléliques sont connues pour chaque individu
iii. Ajouter un nouvel individu à partir de ce panel de départ, dans lequel l'individu ajouté est choisi de manière à augmenter le plus possible le nombre de clusters de génotypes qui répondent à la condition souhaitée (au moins n individus par groupe de génotype).
iv. Répéter l'étape (c) jusqu'à ce que l'on obtienne au moins n individus par cluster de génotype.

**5.** Méthode selon la revendication 4, dans laquelle les individus du panel de référence ont été génotypés plus d'une fois avec toujours le même résultat ou sont la descendance de parents dont le génotype est connu.

**6.** Méthode selon la revendication 4 ou 5, dans laquelle le nombre minimal n d'individus devant être présents dans chaque cluster de génotype est de 5.

**7.** Méthode selon l'une des revendications 1 à 6, comprenant en outre la sélection de marqueurs qui peuvent être utilisés pour le génotypage des individus en :

(a) introduisant des données brutes obtenues dans un programme informatique d'analyse des données de génotypage de biopuces, dans lequel ledit programme informatiques calcule, pour chaque marqueur utilisé au cours de l'exécution, des clusters, chaque individu étant assigné à un cluster pour chaque marqueur
(b) calculant, pour chaque marqueur, des indicateurs représentant la fiabilité du marqueur,

un marqueur étant sélectionné en vue d'une utilisation future pour le génotypage d'individus si les indicateurs sont supérieurs à un seuil prédéterminé, et
dans lequel lesdits indicateurs sont

(a) HomDiff loc = pour chaque locus, le pourcentage d'individus pour lesquels l'allèle déterminé est homozygote et l'allèle attendu est homozygote mais inversé [avec l'autre génotype [allèle]].
(b) HetDiff loc = pour chaque locus, le pourcentage d'individus pour lesquels l'allèle déterminé est homozygote et l'allèle attendu est hétérozygote ou pour lesquels l'allèle déterminé est hétérozygote et l'allèle attendu est homozygote.

**8.** Méthode selon la revendication 7, dans laquelle lesdits indicateurs sont *DensityAA* correspondant à la concentration de valeurs manquantes entre le cluster homologue "AA" et le cluster hétérologue "AB" ou *DensityBB* correspondant à la concentration de valeurs manquantes entre le cluster homologue "BB" et le cluster hétérologue "AB".

**9.** Méthode selon l'une des revendications 7 à 8, dans laquelle les marqueurs qui peuvent être classés dans l'une des classes suivantes sont considérés comme fiables si :

(a)

(1) MaxDensity<30 OU
(2) 30<=MaxDensity<150 et Dunn>0,03

OU

(b)

(1) un cluster AA, AB ou BB n'existe pas
(2) OU pas de données manquantes entre cluster et dunn>0.1
(3) OU MaxDensity <80

OU
(c)

(1) MaxDensity<30, OU
(2) 30<=MaxDensity<150 et dunn>0,03 et taux d'appel (Call Rate) >95

OU
(d)
dunn>0,1 et MaxDensity <80.

**10.** Méthode selon l'une des revendications 1 à 9, dans laquelle un groupe "Absence de signal" a été attribué à un marqueur utilisé dans le cycle de génotypage, ledit groupe ayant été attribué par une méthode comprenant les étapes suivantes

(i) saisie des données brutes obtenues à partir de plusieurs individus dans un programme informatique d'analyse des données de génotypage de biopuces, dans lequel ledit programme informatique calcule, pour chaque marqueur, des clusters de génotypes, chaque individu étant affecté à un cluster pour chaque marqueur
(ii) pour chaque marqueur

(b1) séparer le cluster hétérozygote AB en deux nouveaux clusters hétérozygotes, de manière à obtenir deux nouveaux clusters et deux médioïdes (un pour chaque cluster).
Condition (1)

(a1) calcul de la valeur HomMin=min(q5%(YAA),q5%(YBB)), q5%(YAA) étant le quartile à 5% de tous les Yi du cluster AA (Yi étant la valeur de l'individu i sur l'axe des Y), et q5%(YBB) étant le quartile à 5% de tous les Yi du cluster BB (Yi étant la valeur de l'individu i sur l'axe des Y),
(c1) calcul de la distance entre le médioïde ayant le plus faible ordonnée et la valeur HomMin
(d1) calcul de la valeur Dist.ABAB= Abs (Ymedoïd1 - Ymedoïd2) avec Ymedoïd1 étant l'ordonnée du médoïde le plus élevé (valeur sur l'axe des Y) et Ymedoïd2 étant l'ordonnée du médoïde le plus bas (valeur sur l'axe des Y).
(e1) calcul de N.ABmin, correspondant au plus petit nombre d'individus dans les deux nouveaux clusters hétérozygotes

Dans laquelle un cluster "Absence de signal" est attribuée au marqueur si la valeur calculée en (c1) est supérieure à un premier seuil prédéterminé, et si la valeur calculée en (d1) est supérieure à un premier seuil prédéterminé et si la valeur calculée en (e1) est supérieure à un premier seuil prédéterminé,
Et
Condition (2)
(a2) calcul de N.NAbelowHomo : nombre d'individus pour lesquels aucun allèle n'a été attribué, et ayant une ordonnée inférieure à HomMin, et une abscisse comprise entre l'absicisse du médoïde du cluster AA et l'abscisse du médoïde du cluster BB.
Dans laquelle un cluster "Absence de signal" est attribué au marqueur si la valeur calculée en (a2) est supérieure à un premier seuil prédéterminé.
Et
Condition (3)

(a3) si
a3i) l'ordonnée d'un médoïde de l'un des clusters hétérozygotes est inférieur à l'ordonnée la plus basse du médoïde des clusters AA ou BB, la distance des ordonnées étant supérieure à un seuil prédéterminé, et
a3ii) la distance entre les deux cluster hétérozygotes (Dist.ABAB) est supérieure à un seuil prédéterminé, et

a3iii) le nombre d'individus dans le cluster hétérozygote le plus bas et qui n'ont été assignés à aucun cluster est supérieur à un seuil prédéterminé,

dans laquelle un cluster "Absence de signal" est attribué au marqueur si toutes les conditions a3i à a3iii sont remplies.
Et
Condition (4)
(a4) Calcul de la valeur HomMin - Min($Y_{medoid1}$, $Y_{medoid2}$) - Dist.ABAB Dans laquelle un cluster "Absence de signal" est attribuée au marqueur si la valeur calculée en (a4) est supérieure à un premier seuil prédéterminé et si le nombre d'individus dans la grappe hétérozygote la plus basse est supérieur à un premier seuil prédéterminé.

(iii) Attribuer un groupe "Absence de signal" au marqueur si l'une au moins des conditions 1) à 4) est remplie.

**11.** Méthode selon la revendication 10, dans laquelle les seuils sont les suivants

Condition 1 :

0,2 pour la valeur calculée en (c1)
0,4 pour la valeur calculée en (d1)
5 pour la valeur calculée en (e1)

Condition 2 :
8 pour la valeur calculée en (a2)
Condition 3

0,2 pour l'ordonnée
0,4 pour la distance entre les deux groupes hétérozygotes
5 pour le nombre d'individus dans le cluster hétérozygote le plus bas et qui n'ont été assignés à aucun cluster

Condition 4

0,2 pour la valeur calculée en (a4) et
5 pour le nombre d'individus dans la grappe hétérozygote la plus basse

**12.** Produit programme d'ordinateur, qui analyse les données brutes d'une série de génotypage, comprenant des instructions pour assigner chaque individu d'une série de génotypage à un cluster de génotype, lorsque ces instructions sont exécutées par un circuit logique ou un processeur, comprenant des paramètres optimisés par une méthode selon l'une quelconque des revendications 1 à 11.

**13.** Méthode mise en oeuvre par ordinateur pour le génotypage des individus d'une série de génotypage, comprenant les étapes suivantes

i. fournir des données brutes provenant des individus à un produit de programme informatique selon la revendication 12,
ii. attribuer des allèles à chaque individu de la série de génotypage
iii. récupération des informations de génotypage pour chaque individu de la série de génotypage.

**14.** Méthode selon la revendication 13, dans laquelle les données brutes des individus du panel de référence utilisé pour l'optimisation du programme informatique sont fournies avec les données brutes des individus de la série de génotypage.

**15.** Méthode selon la revendication 14, dans laquelle un signal de mauvaise qualité des données brutes de la série de génotypage est détecté si la seule assignation claire des allèles est obtenue pour les données du panel de référence.

**16.** Dispositif informatique, comprenant un circuit logique, comprenant des paramètres optimisés par la méthode selon l'une des revendications 1 à 11 et exécutant des instructions pour assigner chaque individu d'une série de génotypage à un groupe de génotypes, connecté à un dispositif d'interface homme/machine de manière à exécuter la méthode selon l'une des revendications 13 à 15.

**Optimize settings of genotyping software**
- Use panel of reference
- Vary settings
- Choose settings with best output data corresponding to the panel of reference

↓

**Optimize set of markers by selecting reliable markers**
- Use panel of reference
- Use new indicators *HomDiff_loc* and *HetDiff_loc*

↓

**Perform genotyping run**
- input raw data of the test individuals and of the reference panel
- Use the settings as determined above
- Use the marker set as optimized above

↓

**Determine PAV markers**
- Split heterologous clusters in two clusters
- Check whether the inferior cluster corresponds to a PAV cluster, using indicators

↓

**Assign alleles to each individual**

↓

**Check consistency with quality indicators**

# Figure 1

**Figure 2**

**Figure 3**

A.

B.

# Figure 4

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIN et al.** *Bioinformatics,* 2008, vol. 24 (23), 2665-2671
- **ROUSSEEUW.** Silhouettes: a Graphical Aid to the Interpretation and Validation of Cluster Analysis. *Computational and Applied Mathematics,* 1987, vol. 20, 53-65
- **DAVIES ; BOULDIN.** A Separation Cluster Measure. *IEEE Transactions on Pattern Analysis and machine Intelligence PAMI,* 1979, vol. 1 (2), 224-227
- **TERESA A. WEBSTER ; ALIA PIRANI ; MEI-MEI SHEN ; LAURENT BELLON ; HONG GAO.** *Statistical methods for off-target variant genotyping is Affymetrix'Axiom® Arrays*
- **REYNOLDS, A. ; RICHARDS, G. ; DE LA IGLESIA, B. ; RAYWARD-SMITH, V.** Clustering rules: A comparison of partitioning and hierarchical clustering algorithms. *Journal of Mathematical Modelling and Algorithms,* 1992, vol. 5, 475-504